# EUROPEAN PATENT APPLICATION

(11) **EP 4 361 252 A1**
(43) Date of publication of application: **01.05.2024**
(21) Application number: 22828719.9
(22) Date of filing: 21.06.2022
(51) Int. Cl.: C12N 1/20, B01D 15/36

(54) **COMMERCIAL PURIFICATION METHOD FOR HIGH-PURITY BACTERIAL EXTRACELLULAR VESICLES**

(30) Priority: 21.06.2021 KR 20210080108
(71) Applicant: Rosetta Exosome Co., Ltd., Seongnam-si, Gyeonggi-do 13494 (KR)
(72) Inventor: LEE, Chang Jin, Seoul 05378 (KR); SONG, Sung Hyun, Seongnam-si, Gyeonggi-do 13421 (KR); KIM, Si Won, Seongnam-si, Gyeonggi-do 13466 (KR); LEE, Tae Ryong, Yongin-si, Gyeonggi-do 16908 (KR)
(74) Representative: Gassner, Birgitta
(86) International application number: PCT/KR2022/008791
(87) International publication number: WO 2022/270872

(57) **Abstract**

The present invention relates to a method for mass purifying high-purity bacterial extracellular vesicles and, more specifically, the present invention relates to a method for quickly and conveniently isolating and purifying high-purity bacterial extracellular vesicles from a large amount of bacterial cell culture product by means of calcium or cobalt. The purification method of the present invention is appropriate for obtaining high-purity bacterial extracellular vesicles in a commercial scale by treating a large amount of bacterial cell culture product and, particularly, when using calcium which is innocuous to the human body, is more advantageous in purifying bacterial extracellular vesicles to be used in a drug for human body.

## Description

### TECHNICAL FIELD

The present invention provides an efficient method for purifying bacterial extracellular vesicles in a high purity and a large scale. Concretely, the present invention is related to a method for rapidly and conveniently isolating and purifying bacterial extracellular vesicles in a high purity from a large amount of bacterial cell culture using calcium cations or cobalt cations.

### BACKGROUND ART

All cells including bacteria are known to naturally secrete extracellular vesicles. Bacterial extracellular vesicles are vesicles with sizes ranging approximately 10 nm to 1,000 nm and comprise of various biologically active substances from bacteria, such as proteins, lipids, genetic materials (e.g., DNA and RNA), and virulence factors. Bacterial extracellular vesicles can be classified into extracellular vesicles derived from Gram-negative bacteria and those derived from Gram-positive bacteria, and those secreted from Gram-negative bacteria are also known as outer membrane vesicles (OMVs). Bacterial extracellular vesicles are known to function as information carriers such as delivering proteins or genetic materials among the same species, to contribute to killing competing bacteria, enhancing bacterial survival, and to regulate the pathogenesis of infectious diseases caused by bacteria by delivering toxins to the host.

Recently, it has been reported that various bacterial extracellular vesicles have direct therapeutic efficacy against various diseases including cancers, and also act effectively as drug carriers to treat these diseases (Korean Patent Registration No. 10-1752506). In addition, bacterial extracellular vesicles are investigated to be utilized as vaccines to prevent or treat infectious diseases caused by pathogenic bacteria, such as meningitis. As these bacterial extracellular vesicles are not living cells, they can be stored and transported for a long time, and there are low risk of infection.

As it becomes more likely to utilize bacterial extracellular vesicles for various purposes including treating diseases, it is necessary to develop a method for obtaining a large amount of bacterial extracellular vesicles from bacterial cell culture. However, bacterial extracellular vesicles are as small as nanometers in size, and the bacterial cell culture does not only contain extracellular vesicles, but also numerous microscopic substances including other protein particles (e.g., flagellin protein particles) or nucleic acid particles which have similar density, mass, size, or charge with extracellular vesicles. This makes it difficult to purely isolate bacterial extracellular vesicles from a large amount of bacterial cell culture while preserving the vesicular structure and function.

Current techniques to isolate bacterial extracellular vesicles make use of differences in density, particle size, and immunoaffinity. For examples, ultracentrifugation (Momen-Heravi et al., Methods Mol. Biol., 1660: 25-32, 2017), size exclusion (Gamez-Valero et al., Sci. Rep. 6: 33641, 2016), immunoaffinity isolation, microfluidics chip technology and polymer precipitation (Niu et al., PLoS ONE, 0186534, 2017) are being used.

Density gradient isolation is an isolation method using the density of liquid being around 1.0 while the density of extracellular vesicles is around 1.13 to 1.19. Typically, ultracentrifugation is most widely used and most reliable due to simple principles. However, it requires a lot of labor and time due to complicated isolation steps, needs expensive equipment, up to only 0.6 L of fluid can be processed at one time, and the yield and purity are significantly low, and, thus, it is not suitable for obtaining a large amount of bacterial extracellular vesicles.

Size exclusion is a method for filtrating extracellular vesicles using a filter whose pore size is smaller or similar to that of bacterial extracellular vesicles. As it does not require reaction time, extracellular vesicles can be isolated rapidly with a high purity. However, the yield after isolation is low because significant portions of extracellular vesicles adhere to the filter or are filtrated out.

Immunoaffinity isolation is a method for isolating extracellular vesicles by attaching antibodies to bacterial extracellular vesicles, and is highly specific. However, making antibodies takes a long time and is expensive, so it is not quite practical.

Polymer precipitation is a method of eluting and precipitating bacterial extracellular vesicles by adding a hydrophilic polymer such as polyethylene glycol (PEG) to reduce the solubility of bacterial extracellular vesicles. Using this method, bacterial extracellular vesicles can be isolated with low centrifugal force. However, protein contamination can occur by added polymers, due to co-precipitation of proteins present in the sample solution.

Currently, a Gram-negative bacterial OMV isolation kit is commercially available (ExoBacteria^{™} OMV kit; SBI) . This kit utilizes OMV affinity resin column. However, this kit is not suitable for mass production, as it can process only 30 mL at one time.

Therefore, it is necessary to develop a simple method for isolating bacterial extracellular vesicles in a large scale. It is urgent to develop a method for efficiently isolating and purifying bacterial extracellular vesicles with high purity from other bacterial protein particles (e.g., flagellin particles) or nucleic acid particles while preserving vesicular structures and functions.

### PRIOR ART DOCUMENTS

### PATENT DOCUMENTS

(Non-Patent Document 0001) Momen-Heravi et al., Methods Mol. Biol., 1660: 25-32, 2017
(Non-Patent Document 0002) Gamez-Valero et al., Sci. Rep. 6: 33641, 2016
(Non-Patent Document 0003) Niu et al., PLoS ONE, 0186534, 2017

### SUMMARY OF INVENTION

### TECHNICAL PROBLEM

An object of the present invention is to provide a method for simply and rapidly isolating and purifying bacterial extracellular vesicles in a high purity by processing a large amount of bacterial cell culture.

Another object of the present invention is to provide a method for efficiently isolating bacterial extracellular vesicles in a high purity, solving the problem of co-isolation of protein particles or nucleic acid particles along with bacterial extracellular vesicles when using the current isolation techniques.

### TECHNICAL SOLUTION

The present invention has been completed based on unexpected research results showing that calcium cations or cobalt cations enable the purification of bacterial extracellular vesicles in a high purity, and are useful for mass processing.

In the present invention, bacterial extracellular vesicles and calcium cations or cobalt cations form an insoluble complex when bacterial cell culture was treated with calcium cations or cobalt cations. Calcium cations or cobalt cations effectively inhibit co-precipitation of proteins or nucleic acids from bacteria other than bacterial extracellular vesicles, thereby enabling the isolation and purification of bacterial extracellular vesicles from bacterial cell culture in a high purity. The resulting bacterial extracellular vesicle- calcium cations or cobalt cation complex is isolated by various methods such as centrifugation or precipitation by gravity. Then, by detaching calcium cations or cobalt cations from the complex, bacterial extracellular vesicles can be efficiently and simply purified.

The present invention provides a method for purifying bacterial extracellular vesicles in a large scale, comprising the steps of: (a) adding calcium cations or cobalt cations to a bacterial cell culture; (b) forming an insoluble complex by reacting bacterial extracellular vesicles in the bacterial cell culture with calcium cations or cobalt cations; (c) isolating the complex of the bacterial extracellular vesicles and the calcium cations or cobalt cations from the bacterial cell culture; and (d) detaching the calcium cations or cobalt cations from the complex to purify the bacterial extracellular vesicles.

The method of the present invention has no limitations in the processing capacity, allowing a large-scale processing. Therefore, the method of the present invention can simply and rapidly purify a large amount of bacterial extracellular vesicles in a high purity, and can be implemented in a commercial scale.

The term "bacterial extracellular vesicles" in the present invention may refer to as outer membrane vesicles, shedding vesicles, exosomes, ectosomes, microvesicles, microparticles and nanovesicles derived from bacteria, all of which are understood to be included in the scope of the bacterial extracellular vesicles of the present invention. In addition, Gram-negative bacterial extracellular vesicles include outer membrane-derived extracellular vesicles, inner membrane-derived extracellular vesicles, and inner/outer membrane-derived extracellular vesicles (in the form that either outer membrane and inner membrane components exist simultaneously, or the inner membrane is inside the outer membrane, or conversely, the outer membrane is inside the inner membrane), which are also understood to be included in the bacterial extracellular vesicles of the present invention.

The term "bacterial cell culture" in the present invention refers to a culture medium in which bacterial cells (including genetically modified bacteria) are incubated. The culture medium is not particularly limited as long as it is used to incubate bacterial cells, and includes a "natural medium" using natural materials of undefined composition, such as serum and tissue extract, and a "chemically defined medium" manufactured only from substances whose composition and chemical properties are defined. It would be preferred to incubate bacteria in a chemically defined medium to prepare bacterial cell culture, in order to produce bacterial extracellular vesicles with uniform effects. The chemically defined medium that may be used in the present invention may be selected from the group consisting of M9 medium, Dulbecco's modified Eagle's medium (DMEM medium), and Roswell Park Memorial Institute medium 1640 (RPMI 1640 medium), but the present invention is not limited thereto.

The type of bacteria used in the present invention is not particularly limited, and, in the present invention, the term "bacteria" comprises naturally existing Gram-negative and Gram-positive bacteria and genetically modified bacteria.

The genetically modified bacteria include bacteria transformed to attenuate the toxicity of the extracellular vesicles, for example, bacteria with defective or modified endotoxin-producing genes. Preferably, they may be Gram-negative bacteria transformed to attenuate the toxicity of lipopolysaccharide or Gram-positive bacteria transformed to attenuate the toxicity of lipoteichoic acid. More preferably, they may be bacteria transformed such that the gene encoding lipid A acyltransferase (*msbB*), which is a lipid component of lipopolysaccharide, is deleted (Δ*msbB*), and, most preferably, they may be *Escherichia coli* Δ*msbB*, but the present invention is not limited thereto.

In one embodiment of the present invention, the concentration of calcium cations or cobalt cations is 1 to 1,000 mM, preferably 1 to 500 mM, 1 to 100 mM, 5 to 100 mM, 5 to 50 mM, 5 to 20 mM, 5 to 15 mM, or 5 to 10 mM. Particularly preferably, the concentration of calcium cations or cobalt cations is 5 to 20 mM. If the concentration of calcium cations or cobalt cations is less than 5 mM, bacterial extracellular vesicles are not sufficiently precipitated, and, if the concentration of calcium cations or cobalt cations is higher than 20 mM, the yield of bacterial extracellular vesicles does not increase proportionally even if the cation concentration further increases.

In one embodiment of the present invention, the method of adding calcium cations or cobalt cations comprises a method of adding a solution comprising calcium cations or cobalt cations to a bacterial cell culture and a method of adding calcium cations or cobalt cations in a solid form and dissolving the mixture. However, there is no particular limitation as long as it is in a form that it can react with extracellular vesicles in the bacterial cell culture in a cationic state.

Extracellular vesicles specifically bind to cations to form an insoluble extracellular vesicle-cation complex, and the insoluble complex can be easily isolated because it settles due to gravity. However, bacterial cell culture includes a large amount of impurities such as a large number of bacterial protein particles or nucleic acid particles which have similar mass, density, size, or charge with that of bacterial extracellular vesicles. Therefore, the bacterial extracellular vesicles may be easily contaminated with the impurities during precipitation by cations. The impurities include flagellin protein particles or nucleic acid particles derived from bacterial flagella.

However, in the method of the present invention, calcium cations can effectively inhibit co-precipitation of impurities, such as bacterial flagellin or nucleic acid particles, when forming and precipitating an insoluble complex together with bacterial extracellular vesicles.

In one embodiment of the present invention, the method is used in the step (step (c)) of isolating the insoluble complex of bacterial extracellular vesicles and calcium cations or the insoluble complex of bacterial extracellular vesicles and cobalt cations from the bacterial cell culture is not particularly limited as long as it is a method used to isolate insoluble substances from a sample comprising water-soluble substances. Methods such as centrifugation, ultracentrifugation, filtration, ultrafiltration, gravity, dialysis, sonication, density gradient, and size exclusion may be selected, but the present invention is not limited thereto. Preferably, filtration, centrifugation, or ultrafiltration may be used in step (c) of the present invention.

In one embodiment of the present invention, the method used in the step (step (d)) of isolating calcium cations or cobalt cations from the complex to purify bacterial extracellular vesicles is not limited as long as it is a method of separating only bacterial extracellular vesicles from the complex by removing the specific binding state of bacterial extracellular vesicles and calcium cations or cobalt cations. In addition, various methods or conditions that can be understood by those skilled in the art to which the present invention pertains can be applied.

In one embodiment of the present invention, a method of adding a chelating agent to the complex of the isolated bacterial extracellular vesicle and calcium cations or cobalt cations may be used in step (d).

In the present invention, the term "chelate agent" refers to an ion, a molecule, or an atomic group comprising two or more coordination atoms that coordinate with a metal ion to form a stable chelate complex, and it is called tridentrate ligand, tetradentrate ligand, pentadentrate ligand, hexadentrate ligand, etc., depending on the number of coordinated atoms. The chelate agent of the present invention may be at least one selected from the group consisting of iminodiacetic acid (IDA), nitrilotriacetic acid (NTA), tris-(carboxymethyl)ethylenediamine (TED), ethylenediamine, ethylenediamine tetraacetate (EDTA), alkylenediamine triacetic acid, diethylenetriaminepentaacetic acid (DTPA), ethylene glycol-bis(β-aminoethyl ether)-N,N,N',N'- tetraacetic acid (EGTA), phosphoserine, and 1,4,7-triazocyclononane (TACN). However, it is not limited thereto as long as it specifically binds to the calcium cations or cobalt cations used in the present invention and can specifically isolate the calcium cations or cobalt cations from the complex.

In one embodiment of the present invention, a method of changing the pH value of a solution containing a complex of isolated bacterial extracellular vesicles and calcium cations or cobalt cations may be used in step (d).

In one embodiment of the present invention, a method of isolating bacterial extracellular vesicles from the complex by changing the concentration of imidazole, histidine, ethylenediamine tetraacetate (EDTA) or salts in a solution containing a complex of isolated bacterial extracellular vesicles and calcium cations or cobalt cations may be used in step (d). Preferably, a buffer solution of pH 10 or less, 0 to 5 M NaCl, 0 to 2 M imidazole, 0 to 2 M metal chelating agent, or a combination of the aforementioned conditions may be applied to step (d) of the present invention, but the present invention is not limited thereto.

In one embodiment of the present invention, the method of the present invention may further comprise a pre-treatment step of the bacterial cell culture prior to adding calcium cations or cobalt cations to the bacterial cell culture (i.e., prior to step (a)).

The pre-treatment step of the present invention is a step of concentrating the bacterial cell culture to reduce the volume of a sample to be processed in the subsequent purification step. Simultaneously, bacterial cells, protein particles or nucleic acid particles may be removed from the unpurified bacterial cell culture. At least one method may be selected from the group consisting of centrifugation, filtration, ultrafiltration, size exclusion, desalting column chromatography, size- exclusion chromatography, ion exchange chromatography, affinity chromatography, polymer precipitation, salt precipitation, organic solvent precipitation, aqueous two-phase system, and enzyme treatment, but the present invention is not limited thereto. Preferably, centrifugation, filtration, polymer precipitation or salt precipitation may be used in the pre-treatment step, and tangential flow filtration (TFF) is most preferred.

In one embodiment of the present invention, the method of the present invention may further comprise the step of post-treating the purified bacterial extracellular vesicles after step (d).

The post-treatment step of the present invention is a further purification step of the purified bacterial extracellular vesicles. At least one method may be selected from the group consisting of centrifugation, filtration, ultrafiltration, dialysis, sonication, density gradient, size exclusion, desalting column chromatography, size exclusion chromatography, ion exchange chromatography, affinity chromatography, polymer precipitation, salt precipitation, organic solvent precipitation, aqueous two-phase system and enzyme treatment, but the present invention is not limited thereto. Preferably, in the post-treatment step, ultrafiltration, dialysis, size exclusion, size exclusion chromatography, ion exchange chromatography, polymer precipitation or salt precipitation may be used, and ultrafiltration or size exclusion is particularly preferred.

In one embodiment of the present invention, the method of the present invention may further comprise a filtration step for removing protein particles or nucleic acid particles other than bacterial extracellular vesicles before or after the precipitation step with calcium cations or cobalt cations.

In one embodiment of the present invention, the method of the present invention may further comprise an enzyme treatment step for removing nucleic acid particles derived from bacteria before or after the precipitation step with calcium cations or cobalt cations. The enzyme is not particularly limited as long as it is an enzyme that can be used to decompose nucleic acid particles from the bacterial cell culture. For example, benzonase may be used, but the present invention is not limited thereto. In the enzyme treatment step, ion exchange chromatography may be preferably performed in combination with or after the treatment with benzonase.

The method for large-scale purification of bacterial extracellular vesicles of the present invention may be used in combination with current isolation techniques, if necessary, to maximize separation efficiency.

### ADVANTAGEOUS EFFECTS OF INVENTION

The method for large-scale purification of bacterial extracellular vesicles according to the present invention can efficiently remove bacterial protein particles or nucleic acid particles, such as flagellin, and is not exposed to extreme environments during the isolation process so as to purify bacterial extracellular vesicles efficiently while preserving its structure and function.

In addition, the method of the present invention has no limitations on the processing capacity of bacterial cell culture and, thus, can process a large amount of bacterial cell culture to obtain a large amount of bacterial extracellular vesicles. Furthermore, as the method does not require expensive equipment or materials such as antibodies, and can achieve high yield and purity, high-purity bacterial extracellular vesicles can be easily obtained on a commercial scale.

Especially, since calcium cations are harmless to the human body, bacterial extracellular vesicles obtained by the method of the present invention using calcium cations are more favorable to be developed as a drug for human body.

### BRIEF DESCRIPTION OF DRAWINGS

FIG. 1A is the result of total protein staining and Western blot against OmpA and FliC, showing impurities contained in a cell-free bacterial cell culture.
FIG. 1B is the result of HPLC analysis, showing impurities contained in a cell-free bacterial cell culture.
FIG. 2A is the result of total protein staining and Western blot against OmpA and FliC, showing that bacterial EVs can be isolated and purified by precipitation using six types of metal cations and a polymer (PEG).
FIG. 2B is the result of HPLC analysis of bacterial EV precipitates isolated using six types of metal cations.
FIG. 2C is the result of HPLC analysis of bacterial EV precipitates isolated using six types of metal cations and treated with benzonase to remove nucleic acids.
FIGS. 3A and 3B are the results of HPLC analysis of EV precipitates obtained by treating with various concentrations of calcium cations.
FIG. 3C is the result of total protein staining of EV precipitates obtained by treating with various concentrations of calcium cations or PEG.
FIG. 4 shows the results of SEC-HPLC (a), DLS (b) and NTA (c) analysis of bacterial EV obtained by the purification method of the present invention on a 50 liter culture fluid in a pilot scale.
FIG. 5 shows the results of transmission electron microcopy (a), SDS-PAGE (b), Western blot against OmpA (c), and nano-flow cytometry (d) of bacterial EVs obtained in FIG. 4.

### MODE FOR INVENTION

Hereinafter, the present invention will be described in details by the following embodiments. However, the following embodiments are only for illustrating the present invention, and the present invention is not limited thereto.

The present invention can be variously modified and applied within the description of the claims described below and the scope of equivalents interpreted therefrom.

### Example 1: Identification and removal of impurities in a cell-free bacterial cell culture

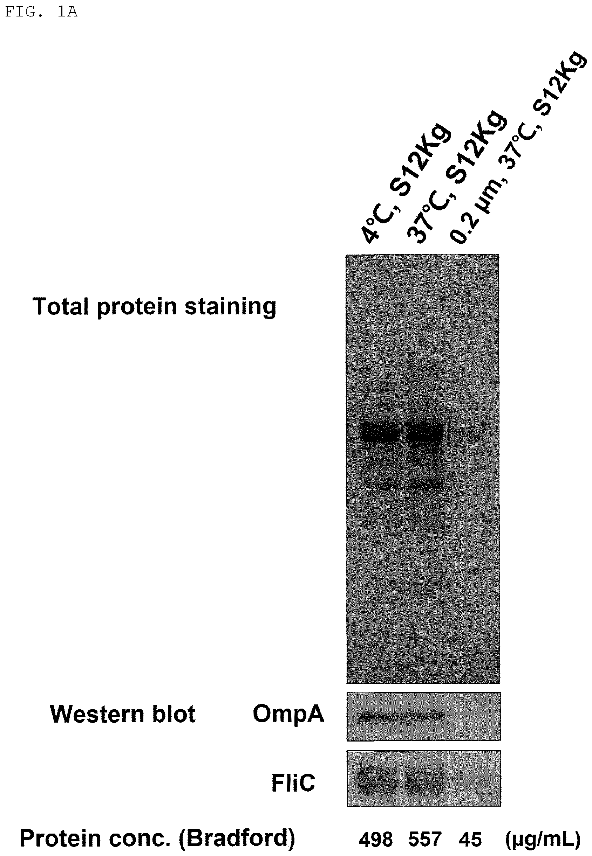

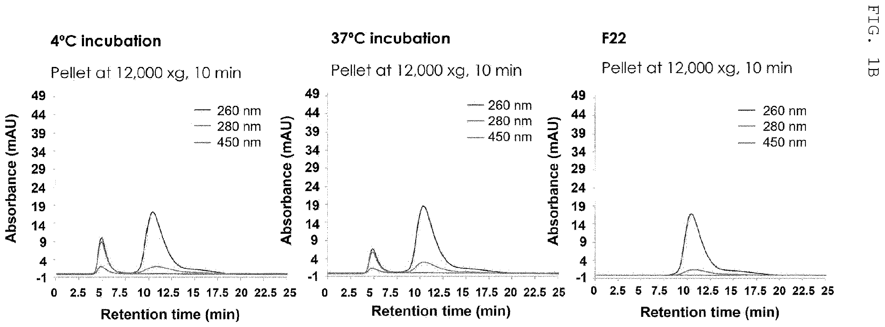

*E. coli* W3110 (*msbB* mutant) culture medium was centrifuged at 6,000 × g for 20 minutes to remove cell pellets, and the supernatant was subjected to tangential flow filtration (TFF) and concentrated 10-fold. The resulting 1 mL of the 10-fold concentrated cell-free cell culture was incubated on ice or at 37 °C for 30 minutes, and then centrifuged (12,000 × g, 10 minutes). Meanwhile, the resulting 1 mL of the 10-fold concentrated cell-free cell culture was filtered through a 0.2 µm pore-sized filter, and the obtained filtrate was centrifuged (12,000 × g, 10 minutes). The pellet obtained after centrifugation was suspended in HEPES-buffered saline (HBS), and then subjected to protein analysis and HPLC analysis. The results are shown in FIGS. 1A and 1B.

To analyze the protein composition pattern, 0.1% SDS was added to each sample of the same volume or protein content, heated at 100°C for 5 minutes for denaturation. Then, each sample was loaded into each well of SDS-polyacrylamide gel (4-20% linear gradient), and subjected to electrophoresis. After reacting the gel with SimplyBlue^{™}(Invitrogen) solution for total protein staining, the total protein pattern of each sample was assessed.

In addition, in order to further analyze the content of FliC and OmpA in the samples, the samples were subjected to SDS-PAGE, and electro-transferred to a PVDF membrane to prepare a blot. After reacting the blot with in-house rabbit polyclonal anti-FliC or anti-OmpA antibodies for more than 4 hours, the blot was then washed and incubated with a secondary antibody (HRP-conjugated anti-rabbit IgG antibody (Santa Cruz)) for 1 hour. After washing the blot, a chemiluminescent substrate (Enhanced chemiluminescence (ECL), Thermo Scientific) was treated to detect luminescent signals of FliC or OmpA.

In addition to the aforementioned analysis, to analyze the chromatogram and the differences in absorbance by wavelength of each sample, multi-wavelength absorption chromatogram was recorded and analyzed using SEC-HPLC system (Ultimate 3000, Thermo Scientific) and a multi-wavelength UV detector. Concretely, Sephacryl S300 (GE Healthcare) size-exclusion chromatography column (10 × 100 mm) was used as the stationary phase. Then, the column was equilibrated using the mobile phase buffer (20 mM HEPES, 500 mM NaCl, pH 7.2) at a flow rate of 0.5 mL/min, and 50 µL of each sample was injected. After injecting the samples, absorption chromatograms were obtained by simultaneously recording the absorbance of eluents at 260 nm, 280 nm, and 450 nm in real time, and the chromatograms were assessed. Various particles including bacterial extracellular vesicles had a peak detected at about 4.9 - 5.2 minutes under the corresponding column and elution conditions, and the peaks that elute later are small non-particulate substances composed of various substances (these non-particulate substances can be easily removed by dialysis, etc.)

Since EVs do not precipitate after centrifugation at 12,000 × g for 10 minutes, impurities contained in the bacterial cell culture could be identified by analysis of the pellet obtained by centrifugation.

As shown in the protein analysis results of the pellet (FIG. 1A), protein bands with various sizes were observed as impurities, and it was confirmed that protein particles comprising OmpA and flagellin (FliC) were contained in large amounts, as shown in Western blot (see first and second lanes of FIG. 1A). Meanwhile, it was confirmed that these impurities were significantly removed when filtered through a 0.2 µm pore-sized filter before centrifugation (see third lane of FIG. 1A).

Similarly, as shown in the HPLC results of these pellets (FIG. 1B), it was confirmed that the particulate impurity peak found around 5 minutes mostly disappeared when filtered through a 0.2 µm pore-sized filter (F22) . That is, most of these particulate impurities contained in the cell-free bacterial cell culture could be removed by filtration through a 0.2 µm filter.

The particulate impurities are very large particles, which may be products of cell death, may be modified/ inactivated EVs. They may precipitate together with EVs during EV isolation by precipitation, and it may be difficult to distinguish them from OmpA-containing EVs. Therefore, it is preferred to remove them through filtration before or after precipitation. In the subsequent examples of the present invention, the impurities were removed before EV precipitation step.

### Example 2: Efficiency of purifying bacterial EVs using various precipitation methods

Efficiency of purifying bacterial EVs was compared using various metal cations (i.e., CA, CO, CU, MN, NI and ZN) and polymers (PEG).

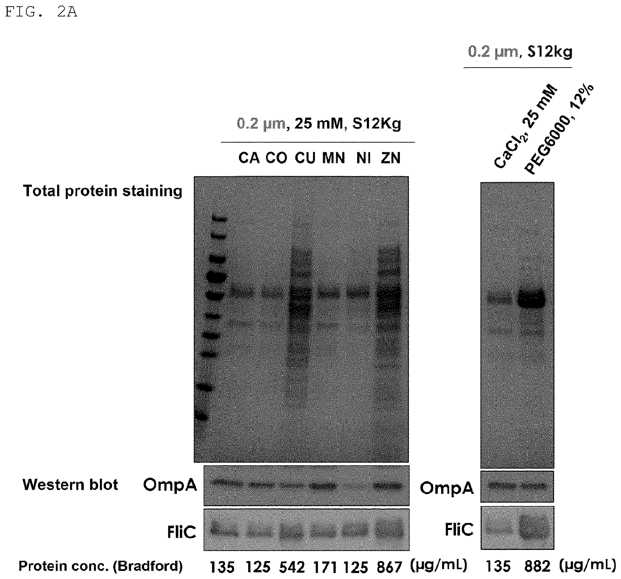

The 10-fold concentrated cell-free cell culture of Example 1 was filtered through a 0.2 µm pore-sized filter, treated with 25 mM of each of six metal cations (i.e., CA, CO, CU, MN, NI, ZN) or 12% PEG6000, incubated for 10 minutes. Then, the pellet was obtained by centrifugation at 12,000 × g for 10 minutes, and the protein analysis and HPLC analysis were performed on the pellet as in Example 1. The results are shown in FIGS. 2A to 2C.

As shown in the result of total protein staining in FIG. 2A, various protein impurities, other than EVs, were co-precipitated in the case of CU and ZN. In the case of PEG, it was observed that many protein impurities were co-precipitated and the amount of FliC impurities was particularly high.

As shown in the result of Western blot in FIG. 2A, OmpA signal was relatively low, implying that EV purification yield is low in the case of NI.

As a result, CA, CO, and MN were superior in terms of the yield of EV purification excluding other impurities.

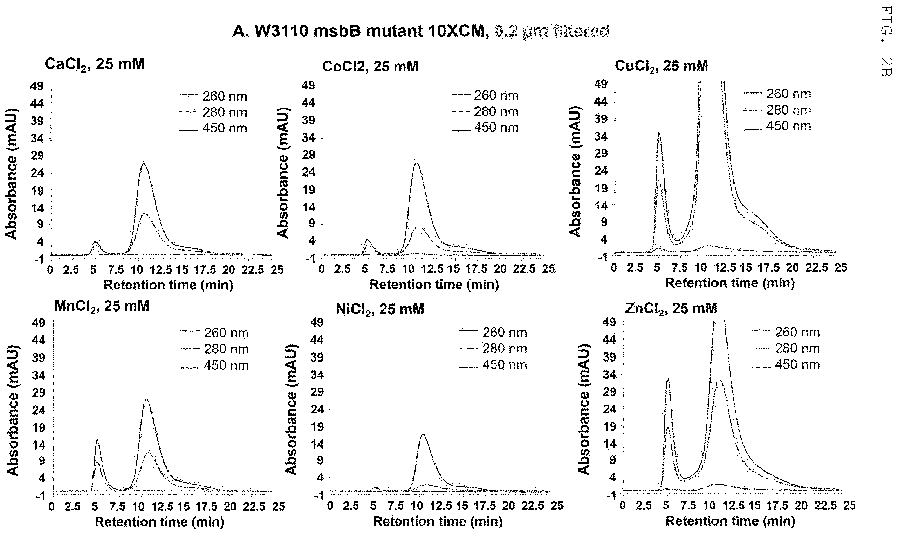

As shown in FIG. 2B, HPLC results also show similar results. The concentration of impurities was relatively low in the case of CA, CO, and MN. Conversely, the peak signal near 5 minutes, corresponding to EVs, was weak in the case of NI.

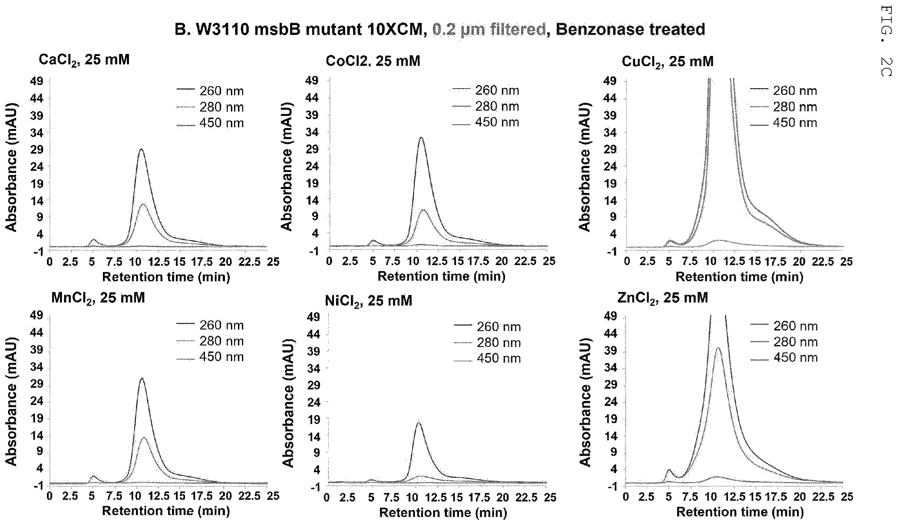

However, when compared with HPLC results after removing nucleic acids by treating with benzonase (FIG. 2C), the resulting pellet obtained using CO, CU, MN, and ZN contained many nucleic acid particles before treatment with benzonase.

Finally, when precipitation was performed using calcium (CA), EVs were obtained most efficiently while most effectively removing the impurities including protein particles (e.g. flagellin) and nucleic acid particles.

### Example 3: Efficiency of purifying bacterial EVs according to calcium concentration

Efficiency of purifying bacterial EVs was compared using various concentrations of calcium cation (CA).

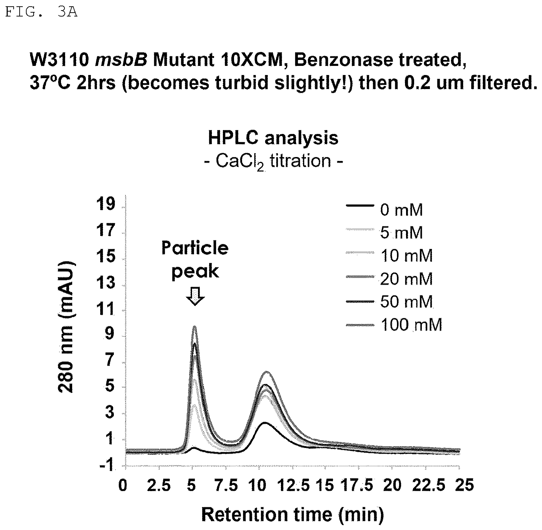

The 10-fold concentrated cell-free cell culture of Example 1 was filtered through a 0.2 µm pore-sized filter, treated with various concentrations of calcium cations, incubated for 10 minutes, and centrifuged at 12,000 × g for 10 minutes to obtain a pellet. The pellet was subjected to the protein analysis and HPLC analysis as in Example 1. The results are shown in FIGS. 3A to 3C.

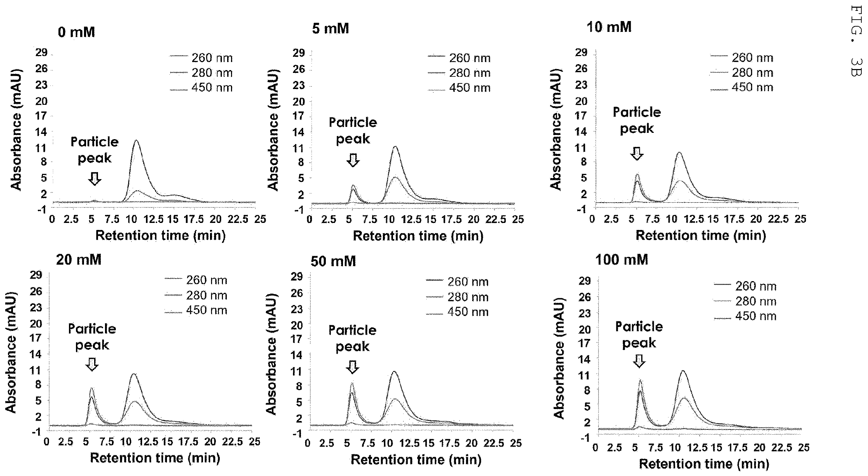

As shown in the HPLC results in FIGS. 3A and 3B, when the calcium ion concentration increased from 5 to 100 mM, EVs were precipitated even at 5 mM, the lowest concentration tested. As the calcium concentration increased, more EVs were precipitated, but the amount of isolated EVs did not increase proportionally above 20 mM.

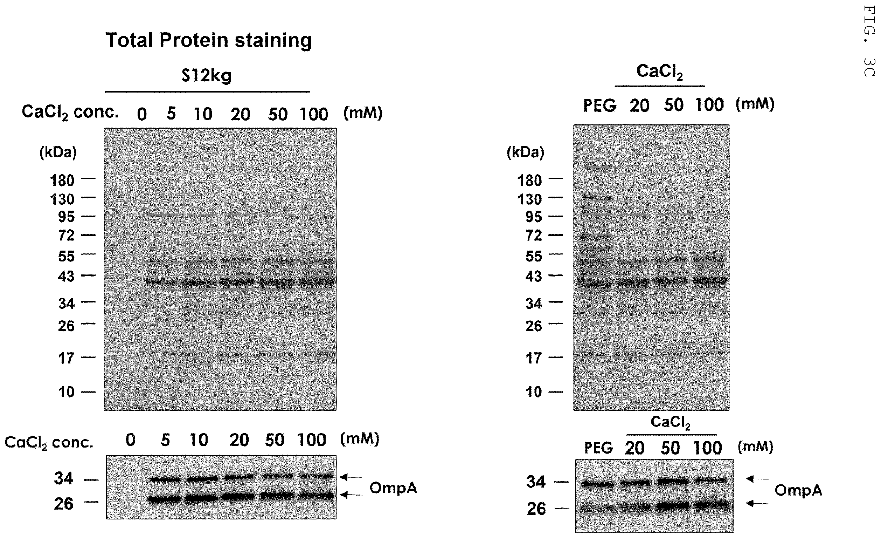

The total protein analysis results in FIG. 3C confirmed that there were little changes in the total protein pattern even when the calcium concentration increased to 100 mM (left panel in FIG. 3C). The type and amount of protein impurities, which were precipitated together with EVs, were significantly reduced when compared with those resulting from PEG precipitation (right panel in FIG. 3C)

### Example 4: Mass production of bacterial EVs

To purify bacterial EVs in a large scale, *E. coli* BL21(DE3) Δ*msbB* strain was cultured under aerobic conditions for 16 hours using a 75-liter Fermenter system and centrifuged at 6,000 × g for 20 minutes to prepare cell-free culture. The culture was filtered through a 0.2 µm pore-sized filter, then the filtrate was subjected to tangential flow filtration using a membrane filter with molecular weight cut-off (MWCO) of 100 kDa to produce a 10-fold concentrated culture. The concentrated culture was treated with 50 µM phenylmethylsulfonyl fluoride (PMSF), and incubated for 30 minutes with shaking to inactivate proteases. The concentrated culture was again filtered through a 0.2 um pore-sized filter, treated with 2 mM MgSO₄ and benzonase (250 U/100 mL) and incubated at room temperature for 1 hour with shaking to remove impurity particles. The culture was treated with a buffer (pH 7.2) containing 25 mM CaCl₂, and incubated under refrigeration for 1 hour with shaking, to selectively precipitate bacterial EVs. The reactant was centrifuged at 13,000 × g for 40 minutes to harvest the precipitate, and the precipitate was dissolved by shaking in a buffer (pH 7.2) containing a chelate agent and filtered through a 0.2 µm pore-sized filter. Then, to exchange non-particulate contaminants with a molecular weight smaller than bacterial EVs and a buffer, the solution was placed in a 100 kDa MWCO dialysis membrane. The dialysis buffer was replaced 5 times for a total of 18 hours at 4°C, and bacterial EVs were further purified.

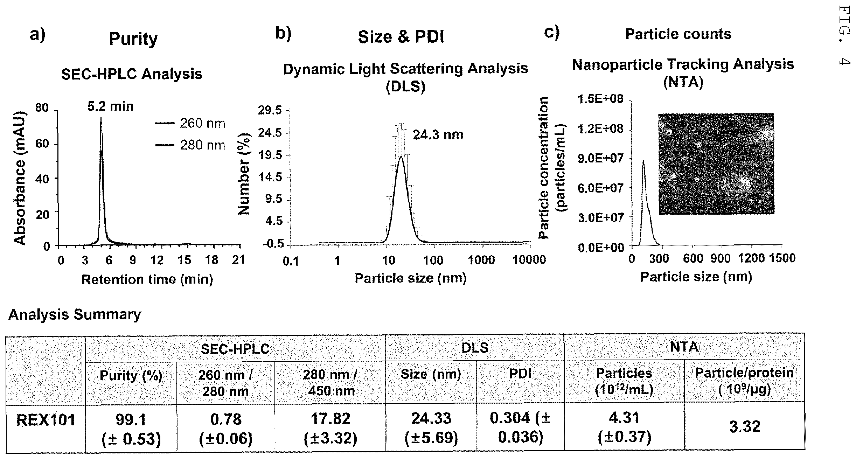

Consequently, a total of 200 mg of bacterial EVs were harvested from 50 liters of the culture (i.e. , 4 mg per 1 liter of the culture) . The bacterial EVs purified by the aforementioned method was analyzed with SEC-HPLC, and they showed high purity (more than 99%) based on absorbance at 260 nm and 280 nm (FIG. 4A) . In addition, through dynamic light scattering (DLS), the bacterial EVs had an average size of 24.3 (±5.09) nm and polydispersity index (PDI), an index of particle uniformity, of 0.304 (+0.036); that is, the EVs consisted of fairly uniform particles (FIG. 4B). Furthermore, through nanoparticle tracking analysis (NTA) and Bradford assay (a quantitative protein analysis), the bacterial EVs had Particle/Protein Index of 3.3; that is, the EVs consisted of a very high level of 3.3 × 10⁹ particles per unit protein (FIG. 4C).

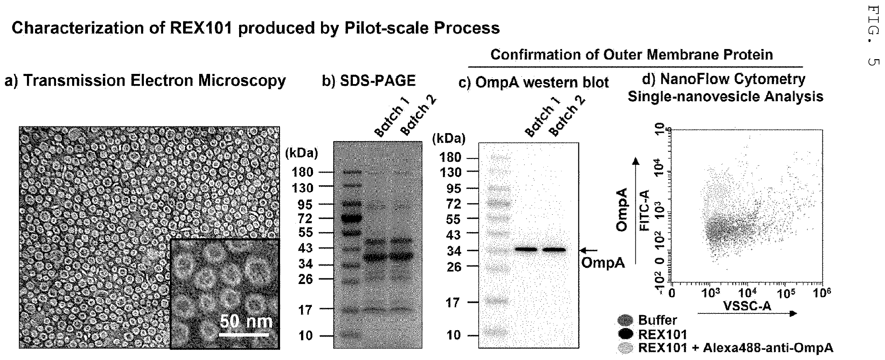

Transmission electron microscopy analysis was also performed to confirm the vesicular and lipid bilayered structure of the purified bacterial EVs. As a result, all the bacterial EVs purified by the aforementioned method had a lipid bilayered structure, and had a uniform spherical vesicular structure (FIG. 5A). Through SDS-PAGE, bacterial EVs secreted from the strain comprised of about 10 major proteins and various minor proteins (FIG. 5B). In addition, Western blot was performed using an antibody against OmpA, the main outer membrane protein of *E. coli,* confirming that OmpA was strongly detected in bacterial EVs purified by the method in the present invention (FIG. 5C). Furthermore, through Nano Flow cytometry analysis, OmpA protein signal was detected in most nanoparticles, confirming that most of the purified bacterial EVs expressed OmpA protein (FIG. 5D).

In this way, when using the calcium precipitation method according to the present invention, it was possible to purify a large amount of bacterial EVs with high yield and purity from a large amount of bacterial culture without the use of expensive equipment or materials such as antibodies. In addition, since purified EVs are not exposed to extreme environments, bacterial EVs can be effectively purified while preserving their structure and function.

## Claims

1. A method for large-scale purification of bacterial extracellular vesicles, comprising the steps of:
(a) adding calcium cations or cobalt cations to a bacterial cell culture;
(b) reacting bacterial extracellular vesicles contained in the bacterial cell culture with the calcium cations or cobalt cations to form insoluble complex;
(c) isolating the complex of the bacterial extracellular vesicles and the calcium cations or cobalt cations from the bacterial cell culture; and
(d) isolating the calcium cations or cobalt cations from the complex to purify the bacterial extracellular vesicles.

2. The method of claim 1, wherein the concentration of the calcium cations or cobalt cations is 1 to 1,000 mM, 1 to 500 mM, 1 to 100 mM, 5 to 100 mM, 5 to 50 mM, 5 to 20 mM, 5 to 15 mM, or 5 to 10 mM.

3. The method of claim 2, wherein the concentration of calcium cations or cobalt cations is 5 to 20 mM.

4. The method of claim 1, wherein step (c) is conducted by one or more methods selected from the group consisting of centrifugation, ultracentrifugation, filtration, ultrafiltration, gravity, dialysis, sonication, density gradient, and size exclusion.

5. The method of claim 1, wherein step (d) is conducted by one or more methods selected from the group consisting of adding a chelate agent, changing a pH value, or changing the concentration of imidazole, histidine, ethylenediamine tetraacetate (EDTA) or a salt.

6. The method of claim 5, wherein the chelate agent is one or more selected from the group consisting of iminodiacetic acid (IDA), nitrilotriacetic acid (NTA), tris-(carboxymethyl)ethylenediamine (TED), ethylenediamine, ethylenediamine tetraacetate (EDTA), alkylenediamine triacetic acid, diethylenetriaminepentaacetic acid (DTPA), ethylene glycol-bis(β-aminoethyl ether)-N,N,N',N'-tetraacetic acid (EGTA), phosphoserine, and 1,4,7-triazocyclononane (TACN).

7. The method of any one of claims 1 to 6, wherein the method further comprises a pre-treatment step of the bacterial cell culture prior to step (a), further comprises a post-treatment step of the purified bacterial cell culture after step (d), or comprises both of the pre-treatment and post-treatment steps.

8. The method of claim 7, wherein the pre-treatment step is conducted by one or more methods selected from the group consisting of centrifugation, filtration, ultrafiltration, size exclusion, desalting column chromatography, size exclusion chromatography, ion exchange chromatography, affinity chromatography, polymer precipitation, salt precipitation, organic solvent precipitation, aqueous two-phase system, and enzyme treatment.

9. The method of claim 8, wherein the pre-treatment step is conducted by centrifugation, filtration, polymer precipitation or salt precipitation.

10. The method of claim 9, wherein the pre-treatment step is conducted by tangential flow filtration (TFF).

11. The method of to claim 10, wherein the pre-treatment step is further conducted by polymer precipitation or salt precipitation after tangential flow filtration (TFF).

12. The method of claim 7, wherein the post-treatment step is conducted by one or more methods selected from the group consisting of centrifugation, filtration, ultrafiltration, dialysis, sonication, density gradient, size exclusion, desalting column chromatography, size exclusion chromatography, ion exchange chromatography, affinity chromatography, polymer precipitation, salt precipitation, organic solvent precipitation, aqueous two-phase system and enzyme treatment.

13. The method of claim 12, wherein the post-treatment step is conducted by ultrafiltration, dialysis, size exclusion, size exclusion chromatography, ion exchange chromatography, polymer precipitation or salt precipitation.

14. The method of any one of claims 1 to 6, wherein the method further comprises an enzyme treatment step for removing nucleic acid particles derived from bacteria prior to step (a), after step (b), after step (c), or after step (d).

15. The method of claim 14, wherein the enzyme treatment step is conducted by using benzonase.

16. The method of claim 15, wherein the method comprises the step of performing ion exchange chromatography in combination with or after the treatment with benzonase.
